Europäisches Patentamt

European Patent Office

Office européen des brevets

(19)

(11) Veröffentlichungsnummer: **0 167 877 B1**

# EUROPÄISCHE PATENTSCHRIFT

(12)

(45) Veröffentlichungstag der Patentschrift: **27.12.91**

(21) Anmeldenummer: **85107359.3**

(22) Anmeldetag: **14.06.85**

(51) Int. Cl.5: **A61B 3/12, A61B 3/14, A61B 3/10**

(54) **Gerät zur Darstellungflächenhafter Bereiche des menschlichen Auges.**

(30) Priorität: **14.06.84 DE 3422144**

(43) Veröffentlichungstag der Anmeldung:
**15.01.86 Patentblatt 86/03**

(45) Bekanntmachung des Hinweises auf die
Patenterteilung:
**27.12.91 Patentblatt 91/52**

(84) Benannte Vertragsstaaten:
**AT CH DE FR GB IT LI NL**

(56) Entgegenhaltungen:
**DE-A- 3 245 939**
**FR-A- 2 555 039**
**US-A- 4 170 398**
**US-A- 4 213 678**

**APPLIED OPTICS, Band 19, Nr, 17, 1 September 1980, R.H. WEBB et al. "Flying spot TV opthalmoscope" Seiten 2991-2997**

**THE JOURNAL OF HISTOCHEMISTRY AND CYTOCHEMISTRY, Band 27, Nr. 1, 1979 R. SHACK et al. "Ultrafast Laser Scanner Mikroskope" Seiten 153-159**

(73) Patentinhaber: **Heidelberg Instruments GmbH**
**Im Neuenheimer Feld 518**
**W-6900 Heidelberg 18(DE)**

(72) Erfinder: **Bille, Josef, Prof. Dr.**
**Am Pferchelhang 2/4**
**W-6900 Heidelberg(DE)**

(74) Vertreter: **Lutz, Johannes Dieter, Dr.rer.nat.**
**Eugensplatz 5**
**W-7000 Stuttgart 1(DE)**

## Beschreibung

Die Erfindung betrifft ein Gerät zur Darstellung flächenhafter bzw. dünner, schichtförmiger Bereiche des menschlichen Auges, insbesondere dessen vorderer Abschnitte mit den im Oberbegriff des Patentanspruchs 1 genannten, gattungsbestimmenden Merkmalen.

Ein Gerät dieser Art ist durch die US-PS 4 213 678 bekannt.

Bei dem bekannten Gerät handelt es sich um ein sog. Laser-Ophthalmoskop, mit dem für diagnostische Zwecke geeignete Darstellungen des Augenhintergrundes gewinnbar sind. Mittels eines auf die Retina fokussierten Laser-Lichtbündels wird das Untersuchungsfeld punktweise abgetastet und aus der Intensität des von den einzelnen Punkten des Untersuchungsfeldes reflektierten bzw. gestreuten Lichtes eine Fernsehbild-Darstellung des Untersuchungsfeldes erzeugt.

Um das für die Intensitätsmessung störende Licht des sog. Cornea-Reflexes zu unterdrücken, ist unmittelbar vor einer Sammellinse, die das vom Untersuchungsobjekt zurückgeworfene Licht zum Photodetektor hin konzentriert, ein Umlenkspiegel angeordnet, über den einerseits das Abtastlicht auf den Augenhintergrund geworfen wird und der andererseits einen zentralen Bereich der Sammellinse, nämlich denjenigen Bereich, auf den die Cornea abgebildet würde, in Ausbreitungsrichtung des zurückgeworfenen Laserlichtes gesehen, abdeckt. Dieser Bereich kann unter den vorgegebenen Abbildungsverhältnissen, unter denen einerseits das von der Retina zurückgeworfene Licht von der Sammellinse erfaßt wird und andererseits die Cornea auf den Umlenkspiegel abgebildet wird, relativ klein gehalten werden. Würde das bekannte Gerät seiner charakteristischen optischen Anordnung jedoch zur Messung an vorderen Abschnitten des Auges benutzt werden, so müßte dieser Umlenkspiegel, um eine wirksame Ausblendung des Cornea-Reflexes vermitteln zu können, wesentlich größer sein, damit nur Licht aus dem Untersuchungsbereich durch die zwischen dem Rand der Sammellinse und dem äußeren Rand des Umlenkspiegels verbleibende, im wesentlichen ringförmige Eintrittspupille der Sammellinse hindurchtreten könnte. Bei einem solchen Einsatz des bekannten Gerätes würde aber das Signal-/Rausch-Verhältnis sehr schlecht, da nur noch ein geringer Teil der vom Untersuchungsbereich zurückkommenden Strahlung durch diese ringspaltförmige Eintrittspupille hindurchtreten könnte. Das bekannte Gerät ist daher allenfalls für Untersuchungen am Augenhintergrund, nicht jedoch für Untersuchungen der vorderen Abschnitte des menschlichen Auges, geeignet.

Da jedoch zahlreiche Veränderungen oder Erkrankungen des Auges selbst wie auch Veränderungen des Stoffwechsels zu Veränderungen im Bereich der vorderen Augenabschnitte (Cornea, Vorderkammer und Augenlinse, sowie Kompartimenten derselben) führen können, deren genaue Erfassung eine frühzeitige Diagnose und damit auch Therapie ermöglichen würden, besteht ein ausgeprägtes Bedürfnis an Geräten, mit denen möglichst detailreiche Informationen über die Form und innere Struktur der vorderen Augenabschnitte gewonnen werden können, insbesondere präzise auswertbare bildliche Darstellungen derselben, die z.B. im Hinblick auf die Gestaltung zur Kompensation von Sehfehlern erforderlicher Linsen, z.B.von Kontaktlinsen, unerläßlich erscheinen.

Die zu Untersuchungen im Bereich der vorderen Augenabschnitte üblicherweise benutzte Spaltlampe oder damit äquivalente Untersuchungsgeräte (US-PS 4 170 398) erfordern einerseits zeitraubende und für die zu untersuchende Person störende Manipulationen oder sind auf wenige, spezielle Einsatz- bzw. Diagnosezwecke ausgelegt.

Aufgabe der Erfindung ist es daher, ein Gerät der eingangs genannten Art zu schaffen, das für umfassende Untersuchungen der vorderen Abschnitte des menschlichen Auges geeignet ist, präzise und leicht interpretierbare Bilddarstellungen des jeweiligen Untersuchungsbereiches ermöglicht und in seiner Anwendung auch für die einer Untersuchung unterworfene Person schonend ist.

Diese Aufgabe wird erfindungsgemäß durch ein Gerät mit den im kennzeichnenden Teil des Patentanspruchs 1 genannten Merkmalen gelöst.

Das erfindungsgemäße Gerät zeichnet sich durch zumindest die folgenden vorteilhaften funktionellen Eigenschaften aus:

Die Verwendung eines Mikroskop-Objektivs zur Fokussierung des Laser-Abtast-Lichtbündels erlaubt eine enge Begrenzung der Tiefe des Untersuchungsbereiches und damit eine hochauflösende Unterteilung des Untersuchungsbereiches in dünne, einzeln darstellbare Schichten, aus denen, z.B. Bei vergleichender Betrachtung, eine Vielzahl für diagnostische Zwecke geeigneter Informationen gewinnbar ist. Das erfindungsgemäße Gerät ist in einer im wesentlichen durch die Brennweite des Mikroskop-Objektives bestimmten Tiefe für eine Vielzahl von Untersuchungen der vorderen Abschnitte des menschlichen Auges geeignet.Durch die mittels der konfokalen Anordnung einer Lochblende mit der Brennebene des Mikroskop-Objektivs erzielte, äußerst wirkungsvolle Ausblendung des Cornea-Reflexes wird ein hohes Signal-/Rausch-Verhältnis erzielt, das ein Abtasten des Untersuchungsfeldes mit hoher Abtastfrequenz erlaubt, so daß die für die Diagnose benötigten Bilddaten innerhalb kürzester Zeit, d.h. innerhalb von Bruchteilen einer Sekunde, allenfalls innerhalb weniger Sekunden, gewonnen werden können und eine einer Untersuchung unterworfene Person kaum belastet wird.

Mittels eines gemäß Anspruch 2 vorgesehenen aktiven Spiegels, für den durch die Merkmale des Anspruchs 3 eine vorteilhafte Anordnung im Rahmen einer Strahl-Formungsoptik des Abtaststrahlenganges angegeben ist, kann die Untersuchungsebene im Untersuchungsobjekt um einige hundert μm, bezogen auf eine Mittelage derselben, die mit der passiven, d.h. in nicht angesteuertem Zustand des Spiegels verknüpften Lage der Untersuchungsebene, vor- und zurückversetzt werden. Die Änderung der Brechkraft des aktiven Spiegels erfolgt durch elektrostatische Auslenkung einer verspiegelten Membran, die praktisch rückhaltlos den elektrostatischen Steuerkräften folgen kann.

Ein wesentlicher Vorteil der Verwendung eines solchen aktiven Spiegels besteht darin, daß keinerlei Verrückungen relativ schwerer Linsensysteme erforderlich ist, zu deren für eine Fokusversetzung geeigneten Verschiebung aufwendige elektromechanische Stellantriebe erforderlich wären.

Die gemäß Anspruch 4 vorgesehene Möglichkeit einer Speicherung der für eine Vielzahl z.B. in Schrittweiten von 0,5 μm bis 10 μm gestaffelter Untersuchungsebenen ermittelter Bild-Daten bietet die Möglichkeit, durch eine rechnergesteuerte Ordnung dieser Bild-Daten Schnittdarstellungen des Untersuchungsobjektes in beliebigen Ebenen oder auch gekrümmten Schnittflächen zu erzeugen. Eine hinreichend kleine Schrittweite von Abtastebene zu Abtastebene und eine hinreichend große Kapazität des Bildspeichers vorausgesetzt, repräsentiert dessen Inhalt ein scharfes räumliches Bild des Untersuchungsobjektes, das, rechnergesteuert, aus beliebigen Richtungen und in beliebigen Schnittdarstellungen betrachtet werden kann.

Um mindestens bereichsweise besonders kontrastreiche Darstellungen des Untersuchungsobjektes erzielen zu können, ist es vorteilhaft, wenn, wie gemäß Anspruch 5 vorgesehen, die Intensität des Abtast-Lichtstroms gezielt erhöht werden kann.

Die durch die Merkmale des Anspruchs 6 ihrem grundsätzlichen Aufbau nach umrissene und durch die Merkmale der Ansprüche 7 bis 11 näher spezifizierte, bevorzugte Ausführungsform des erfindungsgemäßen Geräts vermittelt die folgenden bedeutsamen Eigenschaften und Vorteile:

Mittels einer zusätzlichen Abtasteinrichtung, die mit niedrigerer Abtastfrequenz und asynchron zu der für die Gewinnung der Schichtbild-Informationen vorgesehenen Haupt-Abtasteinrichtung arbeitet, kann für einzelne oder mehrere, einzelnen Bereichen des Bildfeldes zugeordneten Bildpunkten oder auch für gleichmäßig über das Bildfeld verteilte Bildpunkte die sog. Punkt-Bild-Funktion ermittelt werden, die die räumliche Verteilung des jeweils von einem Bildpunkt in den Nachweis-Strahlengang zurückgeworfenen Strahlung beschreibt. Aus einer Analyse der Punkt-Bild-Funktion (PS-Funktion) kann zum einen auf Strukturen des jeweils betrachteten Bildelementes geschlossen werden, die kleiner sind als die Wellenlänge des Abtastlichtes und zum anderen festgestellt werden, inwieweit die Abbildungsverhältnisse von den durch die geometrisch-optischen Randbedingungen vorgegebenen Verhältnissen abweichen. Aus dem Vergleich der realen Punkt-Bild-Funktion mit einer für eine ideale Abbildung charakteristischen Punkt-Bild-Funktion können Stellsignale für die Ansteuerung des aktiven Spiegels gewonnen werden, der dadurch so einstellbar ist, daß sphärische oder astigmatische Aberrationseffekte, die durch das Beobachtungsobjekt selbst bedingt sind, d.h. durch Teile desselben, die vom Abtastlicht durchsetzt werden, bevor es in der Untersuchungsebene fokussiert wird, kompensiert werden, wodurch im Ergebnis gleichsam eine "Glättung" der Brennebene des für die Bildfeldabtastung ausgenutzten Mikroskop-Objektivs möglich ist und Bilddarstellungen erzielbar sind, die weitestgehend wirklichkeitsgetreu und damit auch quantitativ exakt auswertbar sind. Dies ist insbesondere wichtig, um topographische Veränderungen der vorderen Augenabschnitte exakt erfassen zu können, z.B. zur Anpassung von Kontaktlinsen an das Auge des Trägers.

In der durch die Merkmale des Anspruchs 12, ggf. in Kombination mit denjenigen des Anspruchs 13 angegebenen Ausgestaltung des erfindungsgemäßen Geräts können zusätzlich oder alternativ zur Bildfeldabtastung auch die Schichtdicken der untersuchten Strukturen gemessen werden. Dies ist besonders vorteilhaft für Schichtdickenmessungen am Tränenfilm, um zuverlässig beurteilen zu können, wie sich z.B. das Tragen von Kontaktlinsen auf den Tränenfilm auswirkt und innerhalb welcher Zeitspannen der Tränenfilm regenerierbar ist.

Durch die Merkmale des Anspruchs 14 angegebene Auslegung des erfindungsgemäßen Geräts, die durch die Merkmale des Anspruchs 15 auf einfache Weise realisierbar ist, hat den Vorteil, daß in Bereichen des Auges vor der Augenlinse mit hoher Intensität des zur Untersuchung ausgenutzten UV-Lichtes gearbeitet werden kann, ohne daß die der Untersuchung unterworfene Person dadurch belastet wird. Dies ist insbesondere wichtig für kontrastreiche Darstellungen der Endothelschicht, die die Cornea gegen die Vorderkammer des Auges absetzt. Da die Augenlinse für die UV-Strahlung nicht durchlässig ist, wird eine Strahlungsbelastung der Retina des Auges zuverlässig vermieden. Gerade in dem ansonsten im klassischen Meßmethoden schwer zugänglichen Bereich der Cornea und der Vorderkammer des Auges kann ein günstig hohes Signal-/Rausch-Verhältnis erzielt werden.

Vorzugsweise in Kombination hiermit können bei weiterer Realisierung der Merkmale der Ansprüche 16 und ggf. 17 auch Lumineszenzmessungen in den vorderen Augenabschnitten durchgeführt werden, die zusätzliche, für diagnostische Zwecke interessante Informationen liefern.

Dasselbe gilt sinngemäß auch für die Gestaltung des erfindungsgemäßen Geräts gemäß den Merkmalen der Ansprüche 18, 19 und 20, die alternativ oder in Kombination realisiert sein können und polarisationsempfindliche Untersuchungen der vorderen Augenabschnitte ermöglichen, wodurch wiederum eine Fülle zusätzlicher Informationen gewinnbar ist.

Das erfindungsgemäße Gerät erlaubt bei insgesamt einfachem Aufbau eine Vielfalt von Untersuchungen, insbesondere der vorderen Bereiche des Auges und kann in einer für den Patienten besonders schonenden Weise benutzt werden, da die erforderlichen Messungen sehr schnell und mit hoher Präzision durchgeführt werden können.

Weitere Einzelheiten und Merkmale der Erfindung ergeben sich aus der nachfolgenden Beschreibung eines speziellen Ausführungsbeispiels anhand der Zeichnung. Es zeigen:

Fig.1   den Strahlengang einer bevorzugten Ausführungsform des erfindungsgemäßen Geräts,

Fig.2   eine schematische Darstellung der Rechenoperationen zur Auswertung von Punkt-Bild-Funktionen und der hieraus gewinnbaren Stellsignale für einen im Rahmen des erfindungsgemäßen Geräts zur Einstellung der Untersuchungsebenen vorgesehenen aktiven Spiegel und

Fig.3   den grundsätzlichen Aufbau einer im Rahmen des erfindungsgemäßen Geräts gemäß Fig.1 einsetzbare polarisationsempfindlichen Nachweisanordnung.

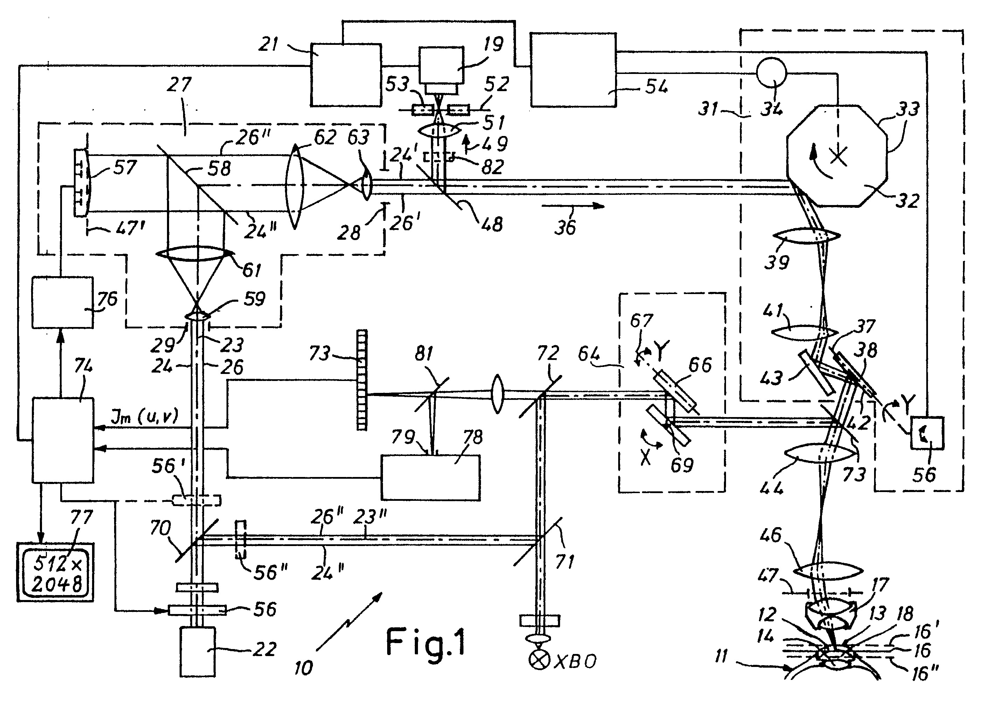

Das in der Fig. 1, auf deren Einzelheiten ausdrücklich verwiesen sei, dargestellte, erfindungsgemäße Gerät 10 ist insbesondere, zur schichtweisen Abbildung des Augenvordergrundes, d.h. der vorderen Abschnitte eines schematisch angedeuteten menschlichen Auges 11 oder Teile derselben für diagnostische Zwecke gedacht. Mit Hilfe dieser Einrichtung 10 sollen Veränderungen z.B. des die Cornea 12 außenseitig umkleidenden dünnen Tränenfilms 13, der Cornea 12 selbst und/oder der Augenlinse 14 oder von Teilbereichen oder -schichten dieser Augenelemente möglichst genau festgestellt werden können, um daraus Rückschlüsse auf Erkrankungen des Auges selbst oder auf Erkrankungen ziehen zu können, die zu Veränderungen im Bereich des Augenvordergrundes führen.

Anhand der nachfolgend beschriebenen konstruktiven und funktionellen Details des Geräts 10 werden auch Verfahrensweisen zu dessen zweckgerechter Benutzung erläutert werden, die ebenfalls als zum Gegenstand der Erfindung gehörig angesehen werden sollen.

Das Gerät 10 ist seinem grundsätzlichen Aufbau nach ein sogenanntes Laser-Abtast-Mikroskop (Laser-Scanning-Mikroskop), das mit zeilenweiser und innerhalb jeder Zeile punktweiser Abtastung des Untersuchungsobjektes oder -bereiches und einer in prinzipieller Analogie zur Darstellung eines Fernsehbildes erfolgenden Darstellung des Untersuchungsbereiches 16, der in der Fig. 1 durch die Fokussierungsebene eines Mikroskopobjektivs 17 schematisch angedeutet ist, die speziell eine Ebene innerhalb der Endothelschicht sei, mittels derer die Cornea 12 an die Vorderkammer 18 des Auges angrenzt.

Dabei werden die einzelnen - im wesentlichen punktförmigen - Elementarbereiche des Untersuchungsbereiches 16 sequentiell in dem genannten Zeilen- bzw. Punktraster mit Laserlicht beleuchtet, und es wird für jeden Elementarbereich die Intensität des von diesem ausgehenden Reflexions- bzw. Streulichtes gemessen, die mittels eines photoelektrischen Detektors, speziell eines Photomultipliers 19 für jedes Bildelement gesondert erfaßt wird. Die solchermaßen erfaßten Intensitätswerte des vom Untersuchungsobjekt zurückgeworfenen Laserlichtes werden synchron zur Lichtabtastung des Untersuchungsobjektes in einen Bildspeicher 21 eingegeben und dort für die weitere Verarbeitung - Bilddarstellung und ggf. rechnerische Verarbeitung der Intensitätsdaten - bereitgehalten.

Als Lichtquelle 22 des Geräts 10 ist ein He-Cd-Laser vorgesehen, der bei den Wellenlängen $\lambda 1$ = 325 nm und $\lambda 2$ = 440 nm, d.h. im nahen UV-Bereich sowie im violetten Bereich des sichtbaren Spektrums Licht emittiert. Dieser Laser 13 ist ein sog. cw-Laser, der kontinuierlich abstrahlt. Der Primärstrahl dieses Helium-Cadmium-Lasers 22 ist in der Fig. 1 durch seinen strich-punktiert eingezeichneten Zentralstrahl 23 und seine Randstrahlen 24 und 26 repräsentiert. Der Primärstrahl 23, 24, 26 des He-Cd-Lasers 22 ist im wesentlichen ein Parallelbündel mit einem Bündelquerschnitt von ca. 1 mm².

Mittels einer insgesamt mit 27 bezeichneten Strahl-Formungsoptik ist eine für die weitere Ausnutzung des Laserlichtes geeignete Bündelform im Sinne einer Anpassung des Bündelquerschnitts an die zur weiteren optischen Verarbeitung vorhandenen optischen Einrichtungen sowie im Sinne einer im folgenden noch näher zu erläuternden Beeinflussung der Konvergenz des Laserlichtbündels einstellbar. Die Randstrahlen des Ausgangslichtbündels der Strahl-Formungsoptik 27, das an deren Ausgang 28 einen größeren Querschnitt hat als an deren Eingang 29, sind mit 24' und 26' bezeichnet.

Das Ausgangslichtbündel 23, 24', 26' der Strahl-Formungsoptik 27 wird einer insgesamt mit 31 bezeichneten Abtast-(Scan)-Einrichtung zugeleitet, die einerseits eine "horizontale" und andererseits eine "vertikale" Strahlablenkung vermittelt, die in dem genannten Abtast-Raster die Abtastung des Untersuchungsbereiches 16 in den X- und Y-Koordinatenrichtungen ermöglicht.

Als Horizontal-Ablenkungs-Element ist ein sog. Polygonspiegel 32 vorgesehen, dessen Facetten 33 in der Darstellung der Fig. 1 ein regelmäßiges Achteck begrenzen. In einer typischen Realisierungsform hat der Polygonspiegel 32 jedoch 24 Facetten 33 in regelmäßigpolygonaler Anordnung zueinander.

Der Polygonspiegel 32 ist mittels eines Elektromotors 34 rotierend antreibbar, wobei die Rotationsfrequenz dieses Polygonspiegels 32 in einer im folgenden noch näher erläuterten Weise mit der Abspeicherung der Ausgangssignale des Photomultipliers 19 in den Bildspeicher 21 synchronisiert ist, in welchem in digitalem Format das Bild des Untersuchungsbereiches 16 abgespeichert wird.

Die Y-Auslenkung des Laser-Lichtbündels 23, 24', 26' ist, in der durch den Pfeil 36 wiedergegebenen Ausbreitungsrichtung des Laser-Lichtbündels 23, 24', 26' gesehen, der X-Auslenkung 32, 34, nachgeordnet, und wird mittels eines um eine horizontale Achse 37 schwenkbar angetriebenen Galvanometerspiegels 38 in für sich bekannter Weise erzielt.

Die Facettenflächen 33 des Polygonspiegels 32 werden in einem sogenannten telezentrischen Strahlengang, der mittels zweier Linsen 39 und 41 realisiert ist, auf die reflektierende Fläche 42 des Galvanometerspiegels 38 abgebildet. Ein zwischen der Austrittslinse 41 dieses telezentrischen Strahlenganges und dem Galvanometerspiegel 38 angeordneter Umlenkspiegel 43 ist lediglich zur Erzielung einer günstigen Strahlengang-Geometrie vorgesehen.

Mittels einer zu der telezentrischen Linsenanordnung 39,41 analogen telezentrischen Linsenanordnung 44, 46, wird die reflektierende Fläche 42 des Galvanometerspiegels 38 auf die Eintrittspupille 47 des Mikroskopobjektivs 17 abgebildet, das seinerseits das als Parallelbündel oder nahezu paralleles Lichtbündel durch die Eintrittspupille 47 hindurchtretende Laserlicht in die Untersuchungsebene 16 fokussiert.

Das an den in der Fokussierungs- bzw. der Untersuchungsebene 16 vorhandenen Strukturen des Untersuchungsobjektes reflektierte bzw. gestreute Laserlicht wird in den insoweit erläuterten Strahlengang zurückgeworfen und mittels eines teildurchlässigen Spiegels 48, in der Regel eines halbdurchlässigen Spiegels, der zwischen dem Polygonspiegel 32 und dem Ausgang 28 der Strahl-Formungsoptik 27 angeordnet ist, in Richtung des Pfeils 49 aus dem Abtast-Strahlengang ausgekoppelt. Dieses ausgekoppelte Lichtbündel 23', 24', 26' wird mittels eines weiteren Mikroskopobjektivs 51 fokussiert; in der Brennebene 52 dieses weiteren Mikroskopobjektivs 51 ist eine Lochblende 53 angeordnet, die eine Bündelbegrenzung dahingehend vermittelt, daß durch diese Lochblende 53 nur solches Laserlicht als für die Intensitätsmessung ausgenutztes Nutzlicht hindurchtreten kann, das aus dem Schärfentiefenbereich des Mikroskopobjektivs 17 entsprechenden Bereich der die Untersuchungsebene 16 bildenden Brennebene des Mikroskopobjektivs 17 stammt. In der Ausbreitungsrichtung 49 des aus dem Beleuchtungsstrahlengang ausgekoppelten Laserlichtes gesehen, ist hinter der Lochblende 53 der Photomultiplier 19 angeordnet, der ein zur Intensität des vom Untersuchungsobjekt in den Abtaststrahlengang zurückgeworfenen Laserlichtes proportionales Ausgangssignal erzeugt.

Die je einem Elementarbereich der Untersuchungsebene 16 zugeordneten Ausgangssignale des Photomultipliers 19 werden, gesteuert durch eine Synchronisiereinrichtung 54, die die zweckgerechte Steuerung der Rotation des Polygonspiegels 32 sowie der Schwenkbewegungen des Galvanometerspiegels 38 vermittelt, letztere durch einen Schwenkantrieb 56, in dem Bildspeicher 21 gespeichert.

Zur Erzeugung eines mittels eines Fernsehbildschirmes darstellbaren Bildes des Untersuchungsbereiches 16 wird das Untersuchungsfeld in 512 x 2 048 Bildpunkte zerlegt, analog zur Erzeugung eines üblichen Fernsehbildes. Es werden demgemäß 512 Bildzeilen abgetastet und innerhalb jeder Zeile 2 048 Bildpunkte. Die Größe, d.h. die Ausdehnung der Bildpunkte in "horizontaler" und "vertikaler" Richtung ergibt sich aus der Größe des Bildfeldes, dividiert durch die Anzahl der Bildpunkte pro Zeile bzw. Bildhöhe dividiert durch die Zeilenzahl. Je nach der Brennweite des Mikroskopobjektivs 17 variiert die Größe des Bildfeldes zwischen 100 µm und mehreren mm; entsprechend variiert die Bildpunktgröße zwischen 0,05 µm und einigen µm.

Die Abtastung des Untersuchungsbereiches 16 erfolgt so rasch, daß das Bildfeld in 40 ms abgetastet wird, wobei in jeweils 20 ms ein Halbbild abgetastet wird, deren eines die ungeraden und der anderes die geraden Zeilenzahlen enthält. Diese beiden Halbbilder werden dann in für sich bekannter Weise einander überlagert.

Die Synchronisierung des Einlesens der Photomultiplier-Ausgangssignale in den Bildspeicher 21 mit den Abtast-Bewegungen des Polygonspiegels 32 und des Galvanometerspiegels 38 erfolgt in bekannter Weise, wie in der wissenschaftlichen Publikation von Scheck (R. Scheck, R. Baker, R. Buchroeder, D. Hillman, R. Shoemaker, and P.H. Bartels, Journal of Histochemistry and Cytochemistry 27, 153 (1979)), beschrieben.

Mit dem Einlesetakt synchronisiert ist auch ein elektrooptischer oder akustooptischer Modulator, mittels dessen die Intensität des in die Untersuchungsebene 16 geworfenen Laserlichts moduliert werden kann. Hierdurch ist es möglich, Bereiche des Untersuchungsfeldes mit höherer Intensität zu beleuchten als den übrigen Teil des Untersuchungsfeldes. Dies ist insbesondere dann von Bedeutung, wenn einzelne Bereiche mit hohem Kontrast abgebildet werden sollen, die Lichtbelastung des Untersuchungsobjektes insgesamt jedoch gering gehalten werden soll.

Um in rascher zeitlicher Folge eine Mehrzahl von Untersuchungsebenen 16, 16' und 16" abtasten zu können, ist ein sog. aktiver Spiegel 57 vorgesehen, dem durch elektrische Ansteuerung eine - positive oder negative - Brechkraft aufprägbar ist, die eine Veränderung der Position der Untersuchungsebene 16, senkrecht zu dieser gesehen, innerhalb eines Bereichs von ± 200 µm, bezogen auf eine vorab gewählte Lage der Untersuchungsebene 16, ermöglicht.

Es sei angenommen, daß dieser aktive Spiegel 57 im nichtangesteuerten Zustand wie ein ebener Spiegel wirkt.

Der aktive Spiegel 57 ist in einer zur Ebene der Eintrittspupille 47 des Mikroskopobjektivs 17 entsprechenden Pupillenebene 47' angeordnet. Er wird über einen teildurchlässigen - halbdurchlässigen - Spiegel 58 der Strahl-Formungsoptik 27 ausgeleuchtet, an dem der mittels einer telezentrischen Linsenanordnung 59,61, die die beiden Sammellinsen 59 und 61 verschiedener Brennweiten umfaßt, aufgeweitete Ausgangslichtstrahlen 23,24,26 des Lasers 22 zum aktiven Spiegel 57 hin umgelenkt wird. Der vom aktiven Spiegel 57 zurückgeworfene, durch die Randstrahlen 26" und 24" repräsentierte, durch den teildurchlässigen Spiegel 58 hindurchtretende Lichtstrom wird mittels einer weiteren telezentrischen Anordnung von Linsen 62 und 63 der Strahl-Formungsoptik 27 auf den für die weitere, bereits erläutere Ausnutzung des Laser-Lichtstromes geeigneten Querschnitt gebracht, mit dem das Laser-Lichtbündel 23, 24', 26', am Ausgang 28 der Strahl-Formungsoptik 27 austritt.

Das insoweit erläuterte Gerät 10 kann somit durch zweckgerechte Ansteuerung des aktiven Spiegels 57, ggf. programmgesteuert, schnell und präzise auf die Abtastung verschiedener, z.B. äquidistant gestaffelter Untersuchungsebenen 16, 16' und 16" eingestellt werden, deren Bilder, eine hinreichende Kapazität des Bildspeichers 21 vorausgesetzt, in ihrer Gesamtheit gespeichert werden können. Der Inhalt des Bildspeichers 21 kann dann zur Darstellung beliebiger Schnittebenen durch den insgesamt erfaßten Untersuchungsbereich ausgenutzt werden, die schräg zu den abgetasteten Untersuchungsebenen oder auch senkrecht zu diesen verlaufen können.

Bezogen auf Darstellungen des vorderen Abschnittes eines Auges können bildliche Darstellungen gewonnen werden, die denselben Informationsgehalt haben wie mit sog. Spaltlampen erreichbare Darstellungen.

Bedingt durch die erläuterte Art der Abtastung der Untersuchungsbereiche 16, 16' und 16" sind die erzielbaren Abbildungen dieser Untersuchungsbereiche zwangsläufig mit Unschärfen behaftet, die dadurch zustandekommen, daß Bereiche, die vor und hinter der Ebene mit optimaler Bildschärfe liegen, zur Intensitätsverteilung des für den Nachweis ausgenutzten Laserlichtes beitragen. Diese Einflüsse können jedoch durch sukzessive Schichtaufnahmen und deren dadurch erfaßbare Veränderungen auch rechnerisch berücksichtigt und kompensiert werden, so daß aus einer rechnerischen Nachverarbeitung des Speicherinhalts scharfe und kontrastreiche Darstellungen der untersuchten Strukturen erzielt werden können.

Dies geschieht zweckmäßigerweise unter Ausnutzung der - als bekannt voraussetzbaren - dreidimensionalen Übertragungsfunktion des Mikroskopobjektivs 17. Die erwähnte rechnerische Korrektur der Bildschärfe anhand einer Serie von Schichtaufnahmen für verschiedene Untersuchungsebenen schließt auch die Möglichkeit ein, ein scharfes räumliches Bild des Untersuchungsobjektes mit gleichsam unendlicher Schärfentiefe erzeugen zu können, da die erwähnte rechnerische Korrektur natürlich für jede Schichtebene erfolgen kann.

Charakteristisches Merkmal des Geräts 10 gemäß Fig. 1 ist, wie schon erwähnt, die konfokale Anordnung der Lochblende 53 zu der die Untersuchungsebene markierenden Brennebene 16 des Mikroskopobjektivs 17, woraus resultiert, daß nur solches Licht durch die Lochblende hindurchtreten kann, das aus dem Schärfentiefenbereich eines durch das Mikroskopobjektiv 53 entwerfbaren Bildes hindurchtreten kann. Die aus anderen Bereichen, die - in Ausbreitungsrichtung des Laser-Lichtes gesehen - vor oder hinter der Brennebene 16 des Mikroskopobjektivs liegen, reflektierten bzw. gestreuten Lichtströme werden durch den konfokalen Strahlengang auf den Rand der Lochblende 53 geworfen und somit gegen den Photomultiplier 19 abgeschattet.

Dies ist insbesondere von Bedeutung für Untersuchungen vorderer Augenabschnitte, die z.B. die an der Innenseite der Cornea 13 angeordnete Endothelschicht betreffen. Diese Endothelschicht hat, da sich ihr Brechungsindex nur geringfügig von dem die angrenzende Vorderkammer 18 des Auges 11 erfüllenden Kammerwasser unterscheidet, ein im Vergleich zum Tränenfilm 13, der die Cornea 12 außenseitig bedeckt, nur geringes Reflexionsvermögen, das etwa 1/100 desjenigen der Cornea beträgt. Trotzdem ist bei dem erfindungsgemäßen Gerät 10 der Beitrag des an dem Tränenfilm 13 reflektierten Lichtes zum Photomultiplier-Ausgangssignal vernachlässigbar, da der Tränenfilm 13, wenn das Gerät z.B. auf die Abtastung der Endothelschicht eingestellt ist, weit außerhalb des Schärfentiefenbereiches des Mikroskopobjektives 17 liegt, wobei der Beitrag des am Tränenfilm 13 reflektierten Lichtes zum Photomultiplier-Ausgangssignal um so geringer ist, je größer der Querschnitt des Laser-Lichtbündels 23, 24', 26' und je kleiner die Brennweite des Mikroskopobjektivs 17 sind.

In typischer Auslegung des Gerätes 10 hat das Mikroskopobjektiv 17 eine Brennweite von 5 mm und eine numerische Apertur von 0,9. Der Durchmesser des Laser-Lichtbündels 23, 24', 26', das durch das Mikroskopobjektiv 17 in die Untersuchungsebene 16 fokussiert wird, beträgt bis zu 6 mm.

Je nach Wahl des Mikroskopobjektives 17 und des genannten Bündelquerschnittes kann ein Auflösungsvermögen von weniger als 0,5 $\mu$m erreicht werden.

Bei Untersuchungen des Tränenfilms 13, der Cornea 12 und/oder der diese gegen die Vorderkammer 18 abgrenzenden Endothelschicht wird mit Licht der kleineren Wellenlänge $\lambda 1$ = 325 nm des He-Cd-Lasers 22 gearbeitet, für das die Augenlinse 14 undurchlässig ist.

Sollen dagegen Strukturveränderungen der Augenlinse 14 untersucht werden, so wird das vom He-Cd-Laser 22 emittierte Licht der Wellenlänge $\lambda 2$ = 440 nm zur Abtastung des Untersuchungsobjektes ausgenutzt.

Bei Untersuchungen mit Licht der Wellenlänge $\lambda 1$ = 325 nm kann mit hoher Intensität gearbeitet und damit ein kontrastreiches Bild z.B. der Endothelschicht entworfen werden, ohne daß hierdurch die lichtempfindliche Retina des Auges 11 belastet wird.

Bedingt durch Inhomogenitäten oder Unregelmäßigkeiten der Schicht des Untersuchungsobjektes, durch die das Laserlicht hindurchtritt, bevor es in der Untersuchungsebene fokussiert wird, ist es möglich, daß die vorstehend stillschweigend als Ebene oder glatte Fläche vorausgesetzte Fokal-"Ebene" Verformungen aufweist, die aus Aberrations- und/oder Astigmatismus-Effekten resultieren. Die Folge solcher Abbildungsfehler wäre ein mindestens bereichsweise unscharfes Bild. Um den Einfluß solcher "Verformungen" der Brennebene kompensieren zu können, ist eine weitere, insgesamt mit 64 bezeichnete Abtast-Einrichtung vorgesehen, die in prinzipiell derselben Weise, wie vorstehend anhand der Abtasteinrichtung 31 erläutert, eine teilweise oder vollständige Abtastung des Untersuchungsbildfeldes ermöglicht. Diese Abtasteinrichtung 64 umfaßt als Abtastelemente einen ersten Galvanometerspiegel 66, der um eine horizontale, d.h. zur Ausbreitungsebene des Abtast-Lichtes parallele Achse 67 schwenkbar ist und die Y-Auslenkung des Abtast-Lichtes vermittelt, sowie einen zweiten Galvanometerspiegel 68, der um eine senkrecht zur Ausbreitungsebene des Abtast-Lichtes verlaufende Achse 69 schwenkbar ist und demgemäß die X-Auslenkung, d.h. die Zeilen-Auslenkung vermittelt.

Als Abtastlicht wird ein vom Ausgangslichtstrom 23, 24, 26, des He-Cd-Lasers 22 abgezweigter Teillichtstrom 23", 24", 26", ausgenutzt. Dieser Teillichtstrom 23", 24", 26" wird in der aus der Fig. 1 ersichtlichen Geometrie über einen ersten, teildurchlässigen Umlenkspiegel 71 und einen zweiten teildurchlässigen Umlenkspiegel 72 sowie über die weitere Abtast-Einrichtung 64 und einen weiteren teildurchlässigen Spiegel 73 in den für die Abbildung des Untersuchungsbereiches 16 vorgesehenen Strahlengang eingekoppelt, wobei diese Einkopplung des weiteren Abtast-Lichtstromes zwischen dem Galvanometerspiegel 38 der ersten Abtast-Einrichtung 31 und der einen Linse 44 der telezentrischen Linsenanordnung 44, 46, erfolgt, die vor der Eintrittspupille 47 des Mikroskopobjektivs 17 angeordnet ist.

Die Intensität des für die zusätzliche Abtastung ausgenutzten Teillichtstromes 23", 24", 26" beträgt nur etwa 10 % der Intensität des Ausgangslichtstromes des He-Cd-Lasers.

Die weitere Abtasteinrichtung 64 wird asynchron zur Abtasteinrichtung 31 und mit wesentlich geringerer Abtastfrequenz betrieben. Das vom Untersuchungsobjekt durch Reflexion bzw. Streuung in den Strahlengang der weiteren Abtasteinrichtung 66 zurückgeworfene Licht Wird mit einer zweidimensionalen (2 D) Matrixanordnung photoelektrischer Empfänger, vorzugsweise einer Dioden-Matrix 73, hinsichtlich seiner zweidimensional-räumlichen Intensitätsverteilung erfaßt, wobei die Intensitätsverteilung der jeweils von einem beleuchteten Bildelement zurückgeworfenen Strahlung gemessen und für eine weitere Verarbeitung gespeichert wird, d.h. es wird die sog. Punktbildfunktion (Point Spread Function - PS-Funktion) aufgezeichnet. Aus der solchermaßen erfaßten PS-Funktion kann unter Anwendung bekannter Algorithmen, deren Grundzüge im folgenden zum besseren Verständnis noch kurz erläutert werden, eine Abweichung des Verlaufs der Wellenfront des durch Teile des Untersuchungsobjekts hindurchtretenden Abtast-Lichtes von dem nach den geometrischoptischen Randbedingungen zu erwartenden Idealverlauf errechnet werden. Insoweit können Abweichungen der PS-Funktion von deren idealem Verlauf schon im Sinne einer Diagnose ausgewertet werden, zumindest als Indiz dafür, daß das Untersuchungsobjekt mit untypischen Veränderungen behaftet ist. Besonders interessant ist die Ermittlung der PS-Funktion deshalb, weil aus der räumlichen Intensitätsverteilung des Punktbildes auf Strukturen geschlossen werden kann, deren charakteristische Dimensionen kleiner sind als diejenigen des als Abtastpunkt beleuchteten Bildelementes.

Des weiteren sind die aus der PS-Funktion entnehmbaren Informationen hinsichtlich des Wellenfront-Verlaufes des Abtast-Lichtes, die auch als "Deformationen" der Brennebene bzw. -fläche des Mikroskopobjektivs 17 interpretiert werden können, zu einer Ansteuerung des aktiven Spiegels 57 im Sinne einer Kompensation solcher Deformationen ausnutzbar. Die mittels der weiteren Abtasteinrichtung 64 gewonnene Bildinformation ist somit als Regelgröße verwendbar, aus deren Überwachung Stellsignale zur Ansteuerung des aktiven Spiegels 57 ableitbar sind, derart, daß dieser die Kompensation von Abbildungsfehlern, die sich in den genannten Deformationen der Wellenfront bzw. der Brennfläche des Mikroskopobjektivs 17 äußern, in genau derjenigen Richtung vermittelt, die durch den mittels der weiteren Abtasteinrichtung 64 abgetasteten Bildpunkt bzw. Bildbereich ausgezeichnet ist.

Diese Auswertung der mittels der weiteren Abtasteinrichtung 64 gewonnenen PS-Funktion wird mittels eines Rechners 74 durchgeführt, der einen Stellsignalgeber 76 ansteuert, der hierauf die für die kompensatorische Einstellung des aktiven Spiegels 57 erforderlichen Ausgangssignale erzeugt, wobei der Rechner 74 auch zur Verarbeitung der im Bildspeicher gespeicherten Intensitäts-Daten zu den möglichen Schicht- bzw. Schnittdarstellungen mittels eines Fernseh-Monitors 77 ausnutzbar ist.

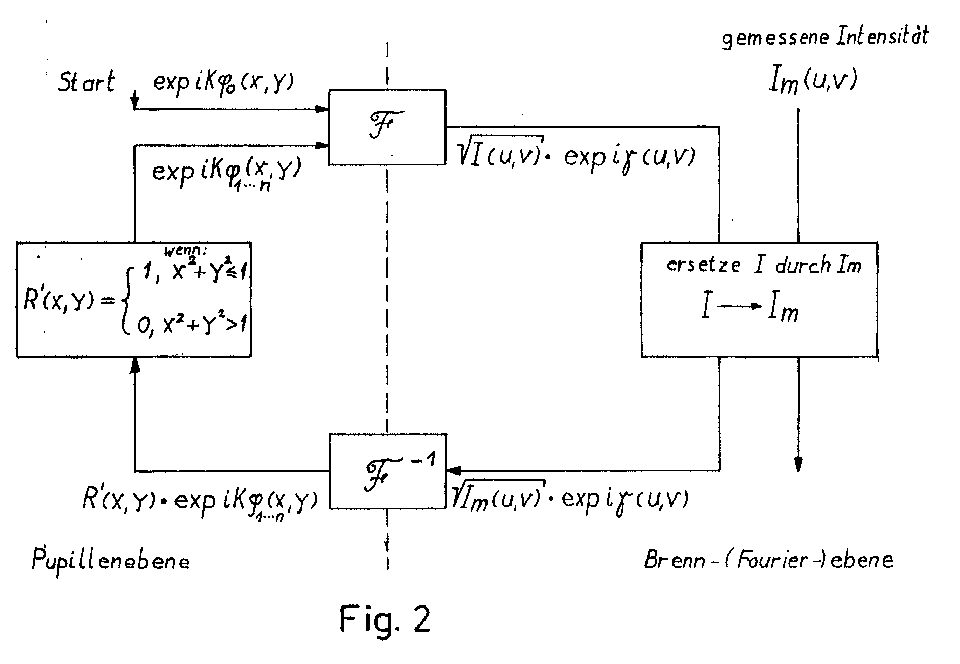

Zu einer vereinfachten Erläuterung des Rechenverfahrens, nach welchem der Rechner 74 die mittels der weiteren Abtasteinrichtung 64 gewonnenen PS-Funktionsdaten verarbeitet, sei nunmehr auf die Fig. 2 verwiesen, in der dieses Rechenverfahren schematisch dargestellt ist:

Dieses Verfahren besteht in der Durchführung einer Iteration, wonach unter Berücksichtigung der mit der Diodenmatrix 73 erfaßten Intensitätsverteilung (der Punktbild- bzw. der PS-Funktion) $I_m (u,v)$ ($u$ = Zeilenindex, $v$ = Spaltenindex der Diodenmatrix) ein Verlauf der Wellenfront des Abtastlichtes am Ort der Eintrittspupille 47 des Mikroskopobjektivs 17 errechnet wird, der mit der gemessenen Intensitätsverteilung $I_m (u,v)$ konsistent ist.

Ausgegangen wird dabei von der Annahme, daß keine Deformationen der Brennebene vorliegen und daß das Lichtfeld am Ort $(x,y)$ der Eintrittspupille 47 des Mikroskopobjektivs 17 eine durch einen Phasenfaktor $\phi_0 (x,y)$ charakterisierbare ebene Wellenfront habe. Unter Berücksichtigung der Übertragungsfunktion des optischen Systems einschließlich einer typischen Struktur des vom Abtastlicht durchsetzten Teils des Untersuchungsbereiches - Faltung der Wellenfront $R(x,y).\exp ik \phi_0 - (x,y)$ mit der Übertragungsfunktion F dieses Systems - wird der Verlauf des Lichtfeldes $\sqrt{I(u,v)}$ .$\exp i \gamma(u,v)$ in der Brennebene - der Untersuchungsebene 16 - des Mikroskopobjektivs 17 berechnet. Durch Ersatz der Amplitudenfaktoren $\sqrt{I(u,v)}$, durch die aus den Messungen gewinnbaren Werte $\sqrt{I_m(u,v)}$ und Anwendung der inversen Übertragungsfunktion $F^{-1}$ auf diesen für die Brennebene angesetzten Lichtfeldverlauf wird nunmehr ein mit diesem Feldverlauf konsistenter Verlauf der Wellenfront an der Eintrittspupille 47 des Mikroskopobjektivs 17, d.h. ein damit konsistenter Phasenfaktor $\phi_1(x,y)$ errechnet. Im ersten Iterationsschritt wird nun dieser Phasenfaktor für die Beschreibung des Lichtfeldes am Ort der Eintrittspupille angesetzt und wieder, wie zuvor beschrieben, das Wellenfeld in der Brennebene des Mikroskopobjektivs 17 errechnet; des weiteren werden die Amplitudenfaktoren durch die Wurzeln der gemessenen Intensitätswerte ersetzt und der neue Phasenfaktor $\phi_2(x,y)$ berechnet. Derartige Iterationsschritte werden so lange wiederholt, bis die gemessenen Intensitäten $I_m(u,v)$ innerhalb vorgebbarer Schranken mit den errechneten Intensitätswerten übereinstimmen und auf diese Weise eine Wellenfront am Ort der Eintrittspupille 47 des Mikroskopobjektivs 17 bestimmt, mit der die gemessenen Intensitätswerte konsistent sind.

Aus dem Vergleich der sich aus dem Iterationsverfahren ergebenden Wellenfront mit der für den Idealfall angenommenen Feldverteilung an der Eintrittspupille 47 des Mikroskopobjektivs 17 können mit Hilfe des Rechners 74 und der Stelleinrichtung 76 Steuersignale zur Ansteuerung des aktiven Spiegels 57 erzeugt werden, derart, daß die gemessene Intensitätsverteilung mit der sich aus einer idealen Feldverteilung am Ort der Eintrittspupille 47 ergebenden Intensitätsverteilung in der Brennebene übereinstimmt.

Auf diese Weise kann die Übertragungsfunktion des optischen Systems so verändert werden, daß die Brennebene 16 des Mikroskopobjektivs 17 gleichsam geglättet wird und die mittels der ersten Abtasteinrichtung 21 gewinnbare Bildinformation einer unter idealen Abbildungsbedingungen gewinnbaren Bildinformation entspricht, mithin ein wirklichkeitsgetreues Abbild des Untersuchungsbereiches gewonnen werden kann.

Im Rahmen des erfindungsgemäßen Geräts 10 ist weiter vorgesehen, daß in dem Strahlengang der weiteren Abtast-Einrichtung 64 auch Test-Licht mit breitbandiger spektraler Verteilung einkoppelbar ist. Dadurch wird die Möglichkeit geschaffen, in bestimmten Bereichen des Beoachtungsfeldes, insbesondere in denjenigen Bereichen, die mittels der "schnellen" Bildabtast-Einrichtung 21 untersucht werden, auch die optischen Dicken der Schichtstrukturen, z.B. des Tränenfilms 13 und/oder der Cornea 12 zu ermitteln. Die Schichtdicke dieser Strukturen wird dabei punktweise erfaßt, wobei bis zu 1 000 Schichtdicken-Werte pro Sekunde gemessen werden können. Die Schichtdickenmessung erfolgt hierbei nach dem Prinzip der sog. Weißlichtinterferenzen, wobei zur Bestimmung der Wellenlängen konstruktiver Interferenz ein Dioden-Zeilen-Spektrometer 78 vorgesehen ist, auf dessen Eintrittsspalt 79 das vom Untersuchungsobjekt zurückgeworfene Licht über einen teildurchlässigen Spiegel 81 eingekoppelt wird.

Als Weißlichtquelle wird zweckmäßigerweise eine XBO-Lampe (Xenon-Hochdrucklampe) verwendet.

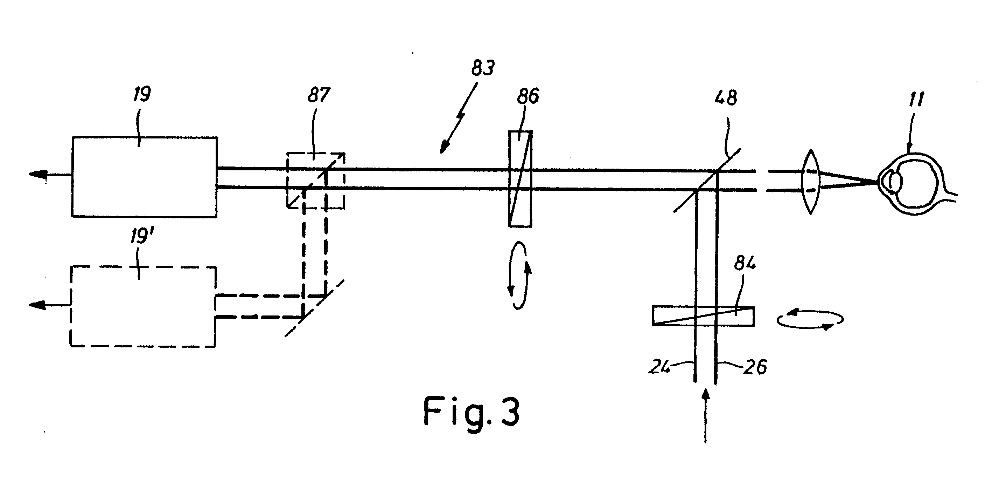

Das Gerät 10 kann dank der Verwendung des He-Cd-Lasers 22 als Lichtquelle auch zur Aufzeichnung der räumlichen Verteilung im Untersuchungsbereich vorhandener lumineszierender Moleküle benutzt werden. Hierzu genügt es, wenn zwischen dem teildurchlässigen Spiegel 48, über dem das für den Nachweis ausgenutzte Licht aus dem Abtast-Strahlengang ausgekoppelt wird, und dem Photomultiplier 19 ein Filter angeordnet wird, das für die anregende UV-Strahlung des Lasers 22 und zweckmäßigerweise auch für dessen im sichtbaren Spektralbereich emittierte Strahlung undurchlässig, für die vom Untersuchungsobjekt ausgehende Lumineszenzstrahlung jedoch durchlässig ist. Die Messung der Lumineszenzstrahlung ist insbesondere interessant für die Bestimmung der Verteilung fluoreszierender Proteine im Kammerwasser. Ein für die Lumineszenzmessung geeignetes Filter 82 wird zweckmäßigerweise, wie in der Fig. 1 gestrichelt angedeutet, zwischen dem teildurchlässigen Spiegel 48 und der Linse 51 angeordnet, in deren Brennebene 52 die Lochblende 53 liegt. Um am Untersuchungsobjekt auch polarisierende Strukturen erkennen und abbilden zu können, ist im Rahmen des erfindungsgemäßen Geräts 10 auch eine in der Fig. 3 wiedergegebene polarisationsempfindliche Detektoranordnung 83 verwendbar, wenn gleichzeitig in dem nur angedeuteten Abtast-Strahlengang 24, 26 mit polarisiertem Licht gearbeitet wird. In spezieller Verfahrensweise wird, um einen definierten Polarisationszustand des ohnehin polarisierten Lasers-Lichtes einstellen zu können, in den Abtast-Strahlengang 24, 26, ein λ/4-Plättchen 84 eingebracht, dessen azimutale Orientierung einstellbar ist. Als Analysator kann ein weiteres λ/4-Plättchen 86 mit ebenfalls einstellbarer azimutaler Orientierung verwendet werden, wobei dieses λ/4-Plättchen 86 zwischen dem Auskoppelspiegel 48 und dem bzw. den zur Intensitätsmessung ausgenutzten Photodetektor(en) 19 und/oder 19' angeordnet ist, dessen bzw. deren intensitätscharakteristische Ausgangssignale in dem Bildspeicher 21 speicherbar sind. Wenn, wie in der Fig. 3 in ausgezogenen Linien dargestellt, nur ein Photomultiplier als Detektor 19 und die beiden λ/4-Plättchen 84 und 86 vorgesehen sind, so werden diese auf denselben Polarisationszustand eingestellt und die mittels des Photomultipliers 19 erfaßte Intensität ist ein Maß für die Häufigkeit von polarisierenden Strukturen, die in der durch den Polarisator 84 und den Analysator 86 ausgezeichneten Orientierung vorliegen. Durch gleichsinnige Veränderung der Einstellung des Polarisators 84 und des Analysators 86 können die verschiedenen Orientierungen solcher polarisierender Strukturen im Untersuchungsbereich erfaßt und abgebildet werden. Alternativ zu dem als Analysator 86 ausgenutzten λ/4-Plättchen kann, wie in der Fig. 3 gestrichelt angedeutet, auch ein polarisationsabhängiger Strahlenteiler 87 in Verbindung mit dem Photomultiplier 19 und einem weiteren Photomultiplier 19' eingesetzt werden, wobei zueinander orthogonale Polarisationskomponenten des vom Untersuchungsobjekt zum polarisationsempfindlichen Strahlenteiler gelangenden Lichtes je einem der Detektoren 19 und 19' zugeleitet werden. Die von den beiden Detektoren 19 und 19' abgegebene Intensitätssignal-Kombination ermöglicht die Identifizierung eines bestimmten Polarisationszustandes und damit, in Kombination mit einem durch Einstellung des Polarisators 24 vorgebbaren Polarisationszustand des Abtastlichtes wiederum die Erkennung der Orientierung polarisierender Strukturen im Untersuchungsobjekt. Eine polarisationsempfindliche Nachweisanordnung ist vorteilhaft, um Strukturen unbekannten Verlaufes, insbesondere im sog. Stroma des Auges, nachweisen und abbilden zu können.

## Patentansprüche

1. Gerät zur Darstellung flächenhafter bzw. dünner, schichtförmiger Bereiche des menschlichen Auges, insbesondere dessen vorderer Abschnitte, mit einer elektrisch steuerbaren optischen Abtast-Einrichtung, die in einem Zeilen- und Spaltenraster, das einer üblichen Fernsehnorm entspricht, eine sukzessive Beleuchtung der Rastereinteilung entsprechender Bildelemente sowie die Messung der Intensität des von den jeweils beleuchteten Bildelementen zu einer photoelektrischen Detektoreinrichtung reflektierten bzw. gestreuten Lichtes vermittelt, und mit einer Einrichtung, die an der Cornea bzw. an dem Tränenfilm reflektiertes Licht aus dem Nachweisstrahlengang ausblendet, wobei im Rahmen der Abtast-Einrichtung als Lichtquelle ein Laser vorgesehen ist, dessen Ausgangslichtstrom auf Brennfleck-Querschnitte von weniger als 0,5 μm Durchmesser fokussierbar ist dadurch gekennzeichnet, daß zur Fokussierung des Laser-Lichtbündels (23, 24', 26') auf den Untersuchungsbereich (16) ein Mikroskop-Objektiv (17) vorgesehen ist, das eine Fokussierung des Untersuchungslichtes auf den Untersuchungsbereich mit einer geringen, der Schichtdicke des Untersuchungsbereiches entsprechenden Schärfentiefe vermittelt, daß der Nachweis-Strahlengang durch einen Teil des Beleuchtungs- bzw. Abtast-Strahlenganges gebildet ist, aus dem mittels einer Strahlenteiler-Anordnung (48) ein Teil des in Richtung auf die Lichtquelle (22) zurückgeworfenen Lichtes für die Intensitätsmessung auskoppelbar ist, und daß in konfokaler Anordnung mit der Brennebene (16) des Mikroskop-Objektivs (17), in Ausbreitungsrichtung des zurückgeworfenen Lichtes gesehen, zwischen der Strahlenteiler-Einrichtung (48) und der Detektoranordnung (19) eine Lochblende (53) vorgesehen ist, durch die nur Licht hindurchtreten kann, das aus dem Schärfentiefenbereich der durch das Mikroskop-Objektiv (17) vermittelten Abbildung stammt.

2. Gerät nach Anspruch 1, dadurch gekennzeichnet, daß der Beleuchtungsstrahlengang einen aktiven Spiegel (57) mit elektronisch steuerbarer Brechkraft umfaßt, durch dessen Ansteuerung der Abstand der Untersuchungsebenen (16,16', 16") von der Hauptebene des Mikroskop-Objektivs (17) veränderbar ist.

3. Gerät nach Anspruch 2, dadurch gekennzeichnet, daß der aktive Spiegel (57) im Rahmen einer zur Anpassung des Bündelquerschnittes des Abtastlichtes an die Eintrittspupille (47) des Mikroskop-Objektivs (17) vorgesehenen Strahl-Formungsoptik (27) mit telezentrischer Eingangsoptik (59, 61) und telezentrischer Ausgangsoptik (62, 63) in einer der Ebene der Eintrittspupille (47) des Mikroskop-Objektivs (17) entsprechenden Pupillenebene (47') angeordnet ist.

4. Gerät nach einem der vorhergehenden Ansprüche, dadurch gekennzeichnet, daß ein mit der Zeilen- und der Spalten-Ansteuerung der Abtastung der Untersuchungsobjekt-Beleuchtung synchronisiert angesteuerter Bildspeicher (21) vorgesehen ist, in dem die für die einzelnen Bildpunkte einer Untersuchungsebene (16; 16'; 16") gemessenen Intensitätswerte des zurückgeworfenen Lichtes für eine Mehrzahl von Untersuchungsebenen speicherbar sind.

5. Gerät nach einem der vorhergehenden Ansprüche, dadurch gekennzeichnet, daß ein elektro-optischer oder akusto-optischer Modulator (56 56', 56") vorgesehen ist, durch dessen mit dem Einlesetakt der Detektor-Ausgangssignale in den Bildspeicher (21) synchronisierte Ansteuerung die Intensität des Abtast-Lichtstromes (23,24',26') veränderbar ist.

6. Gerät nach einem der vorhergehenden Ansprüche, dadurch gekennzeichnet, daß eine zusätzliche Abtasteinrichtung (64) vorgesehen ist, mit der die räumliche Intensitätsverteilung (Punktbildfunktion) der von einem Bildelement des von einem Bildpunkt des Untersuchungsfeldes (16) zurückgeworfenen Lichtes erfaßbar ist.

7. Gerät nach Anspruch 6, dadurch gekennzeichnet, daß eine Vergleichseinrichtung vorgesehen ist, die aus dem Vergleich der gemessenen Intensitätsverteilung mit einer nach der Auslegung der Abbildungsoptik zu erwartenden idealen Intensitätsverteilung Stellsignale für die Ansteuerung des aktiven Spiegels (57) im Sinne einer Kompensation von Abbildungsfehlern erzeugt.

8. Gerät nach Anspruch 6 oder Anspruch 7, dadurch gekennzeichnet, daß zur Bestimmung der Punktbildfunktion (PS-Funktion) eine Anzahl von photoelektrischen Empfängern in Matrix-Anordnung, insbesondere eine 2 D-Diodenmatrix (73) vorgesehen ist, der ein Rechenwerk (74) nachgeschaltet ist, das aus der Verarbeitung der intensitäts-charakteristischen Ausgangssignale der Diodenmatrix (73), die zur Ansteuerung des aktiven Spiegels (57) im Sinne der Kompensation von Abbildungsfehlern erforderlichen Stellsignale erzeugt.

9. Gerät nach einem der vorhergehenden Ansprüche 6 bis 8, dadurch gekennzeichnet, daß das in die zweite Abtast-Einrichtung (64) eingekoppelte Licht mittels eines teildurchlässigen Spiegels (70) vom Ausgangslichtstrom (23,24,26) der Laser-Lichtquelle (22) abgezweigt ist, wobei die Intensität des in die weitere Abtast-Einrichtung (64) eingekoppelten Lichtstromes (23", 24", 26") nur 1/10 bis 1/5 der Intensität des Laser-Ausgangslichtstromes (23,24,26) beträgt.

10. Gerät nach einem der vorhergehenden Ansprüche 6 bis 9,
dadurch gekennzeichnet, daß die Intensität des in die weitere Abtasteinrichtung (64) eingekoppelten Lichtes mittels einer eigenen Modulationseinrichtung einstellbar ist.

11. Gerät nach Anspruch 9 und Anspruch 10,
dadurch gekennzeichnet, daß das in die weitere Abtasteinrichtung eingekoppelte Licht mittels eines zwischen der Lichtquelle (22) und dem ersten Modulator (56') angeordneten teildurchlässigen Spiegels aus dem Ausgangslichtstrom der Lichtquelle (22) auskoppelbar ist, wobei die Modulationseinrichtung der weiteren Abtast-Einrichtung (64) dem teildurchlässigen Spiegel nachgeordnet ist.

12. Gerät nach einem der vorhergehenden Ansprüche in Verbindung mit Anspruch 6,
dadurch gekennzeichnet, daß in die weitere Abtasteinrichtung (64) ein Test-Lichtstrom mit breitbandiger spektraler Verteilung des Test-Lichtes einkoppelbar ist und daß ein Spektrometer (78) vorgesehen ist, mit dem die spektrale Intensitätsverteilung des in den Ausgangszweig der weiteren Abtast-Einrichtung (64) geworfenen Lichtes erfaßbar ist.

13. Gerät nach Anspruch 12, daß das im Weißlicht-Testzweig des Gerätes (10) vorgesehene Spektrometer (78) als Detektoreinrichtung eine Mehrzahl von photoelektrischen Detektoren in Zeilenanordnung vorgesehen sind.

14. Gerät nach einem der vorhergehenden Ansprüche,
dadurch gekennzeichnet, daß die zur Abtastung des Untersuchungsfeldes (16) vorgesehene Lichtquelle (22) mindestens zwei Emissionsbereiche deutlich verschiedener Wellenlangen $\lambda_1$ und $\lambda_2$ hat, wobei der eine Emissionsbereich mit der zentralen Wellenlänge $\lambda_1$ im nahen UV-Bereich (zwischen 300 nm und 400 nm) und der andere Emissionsbereich der zentralen Wellenlänge $\lambda_2$ im sichtbaren Spektralbereich ( $\lambda$ größer 400 nm) liegt.

15. Gerät nach Anspruch 14,
dadurch gekennzeichnet, daß als Lichtquelle (22) ein He-Cd-Laser vorgesehen ist, der Wellenlängen $\lambda_1$ = 325 nm und $\lambda_2$ = 440 nm emittiert.

16. Gerät nach Anspruch 14 oder Anspruch 15,
dadurch gekennzeichnet, daß im Rahmen der Bildfeld-Abtast-Einrichtung (22,31) eine Lumineszenz-Meßeinrichtung vorgesehen ist, mit der alternativ oder gleichzeitig mit der Erfassung des zurückgeworfenen Abtast-Lichtes auch die Verteilung vom Untersuchungsobjekt durch UV-Anregung induzierten Lumineszenz-Strahlung erfaßbar und darstellbar ist.

17. Gerät nach Anspruch 16,
dadurch gekennzeichnet, daß in den zum Detektor (19) der ersten Abtasteinrichtung (31) führenden Zweig des Nachweis-Strahlenganges ein Filter (82) angeordnet ist oder in diesen Zweig des Strahlenganges einführbar ist, das die UV-Komponente des Laser-Lichtes unterdrückt, für die Lumineszenz-Strahlung jedoch durchlässig ist.

18. Gerät nach einem der vorhergehenden Ansprüche,
dadurch gekennzeichnet, daß mindestens ein Polarisations-Stellglied (84) vorgesehen ist, mit dem verschiedene definierte Polarisationszustände des zur Abtastung des Untersuchungsobjektes ausgenutztes Lichtstromes (24,26) einstellbar sind, und daß im Rahmen der Detektoranordnung ein als Analysator ausgenutzer Austrittspolarisator (86; 87) vorgesehen ist, der seinerseits auf einen definierten Polarisationszustand des vom Untersuchungsobjekt zurückgeworfenen Lichtes eingestellt oder einstellbar ist.

19. Gerät nach Anspruch 18,
dadurch gekennzeichnet, daß der Eintrittspolarisator (84) und der Austrittspolarisator (86) auf denselben Polarisationsstand eingestellt bzw. einstellbar sind.

20. Gerät nach Anspruch 18 oder Anspruch 19,
dadurch gekennzeichnet, daß die Detektoranordnung (19, 19') eine für den Polarisationszustand des vom Untersuchungsobjekt zurückgeworfenen Lichtes charakteristische Ausgangssignalkombination erzeugt.

**Claims**

1. Apparatus for imaging planar or thin layer sections of the human eye, particularly their frontal sections, comprising an electrically controllable optical scanner which, in a line and gap raster corresponding to a standard TV format, successively illuminates picture elements corresponding to the raster elements and measures the intensity of the light which is reflected or scattered to a photoelectric detector device from the instantaneously illuminated picture elements, and which comprises a device which stops down the light reflected from the reference beam at the cornea or at the tear film, and in which the light source for the scanner is a laser of which the output light beam is focussable to focal point cross sections of less than 0.5 μm diameter,

characterised in that a microscope objective (17) is provided for focussing the laser-light beam (23,24',26') in the region under examination (16) which enables the illuminating beam to be focussed in the examination region within a small depth of focus equivalent to the layer thickness of the region under examination, the reference beam is formed as part of the illuminating scanning beam, from which a part of the light reflected back in the direction of the light source (22) is divertable by a beam splitter device (48) for measuring its intensity, and that in confocal arrangement with the focal plane (16) of the microscope objective (17) is arranged a diaphragm (53) between the beam splitter device (48) and the detector device (19), viewed in the direction of the reflected light, through which diaphragm (53) can pass only light from the image which is formed within the depth of focus of the microscope objective (17).

2. Apparatus according to claim 1, characterised in that the illuminating beam path includes an active mirror (57) with electronically controllable reflective power, by controlling which reflector the distance of the examination planes (16, 16', 16'',) from principal plane of the microscope objective (17) is variable.

3. Apparatus according to claim 2, characterised in that the active mirror (57) is arranged in an aperture plane (47) corresponding to the plane of the entry aperture (47) of the microscope objective (17) within a beam forming lens system (27) provided for adjusting the beam cross section to the input aperture (47) of the microscope objective (17) and having a telecentric input lens system (59,61) and a telecentric output lens system (62,63).

4. Apparatus according to any of the preceding claims, characterised in that a frame store (21) is provided controlled synchronously with the line and gap raster control of the scanning of the illumination of the object under examination in which the intensity levels of the reflected light measured for the individual image points of a plane (16, 16', 16'') under examination are storable for a plurality of planes under examination.

5. Apparatus according to any of the preceding claims, characterized in that an electro-optic or acoustic-optic modulator (56, 56', 56'') is provided by which the intensity of the scanning light beam (23, 24', 26) is variable by synchronous control with the rate of reading in the detection output signals into the frame store (21).

6. Apparatus according to any of the preceding claims, characterised in that an additional scanner (64) is provided, with which can be determined the spatial intensity distribution (point spread function) of the light reflected from a picture element or an image point of the field (16) under examination.

7. Apparatus according to claim 6, characterised in that a comparator is provided which, by comparing the measured intensity distribution with an ideal intensity distribution to be expected from the construction of the scanning lens system, produces control signals for controlling the active mirror (57) to compensate for imaging defects.

8. Apparatus according to claim 6 or claim 7, characterised in that for determining the point spread function (PS-function) a plurality of photoelectric detections is provided in a matrix arrangement, especially a 2D-diode matrix (73), which is connected to a computer (74) which by processing the output signals of the diode-matrix (73) which are characteristic of the intensities, produces the control signals required for controlling the active mirror (57) for compensating imaging defects.

9. Apparatus according to any of preceding claims 6 to 8, characterised in that light fed into the second scanning device (54) is split by means of a partially transmissive mirror (70) from the output light beam (23,24,26) of the laser light source (22), wherein the intensity of the input light beam (23'',24'',26'') in the further scanning device (64) is only 1/10 to 1/5 of the intensity of the laser output light beam (23,24,26).

10. Apparatus according to any of preceding claims 6 to 9, characterised in that the intensity of the light in the further scanning device (64) is adjustable by means of a separate modulation device.

11. Apparatus according to claim 9 or claim 10, characterized in that light fed into the second scanning device is decoupleable from the output light beam of the light source (22) by means of a partially transmissive mirror arranged between the light source (22) and the first modulator (56'), the modulation device of the further scanning device (64) being connected to the partially transmissive mirror.

12. Apparatus according to any of the preceding claims in combination with the claim 6, characterized in that a test-light beam with a broad band spectral distribution of the test-light can be fed into the further scanning device (64) and a spectrometer (78) is provided with which the spectral intensity distribution of the light diverted into the output branch of the further scanning device (64) may be determined.

13. Apparatus according to claim 12, characterized in that the spectrometer (78) provided in the white light test-branch of the apparatus (10) comprises as a detector device a linear array of a plurality of photoelectric detectors.

14. Apparatus according to any of the preceding claims, characterized in that the light source (22) provided for scanning the field under examination (16) has at least two emission bands of clearly different wavelengths $\lambda_1$ and $\lambda_2$ of which the one emission band of central wavelength $\lambda_1$ lies in the near UV-region (between 300 nm and 400 nm) and the other emission band of central wavelength $\lambda_2$ in the visible region of the spectrum ( $\lambda$ greater than 400 nm).

15. Apparatus according to claim 14, characterized in that an He-Cd-laser is used as the light source (22) which emits wavelengths $\lambda_1 = 325$ nm and $\lambda_2 = 440$ nm.

16. Apparatus according to claim 14 or claim 15, characterized in that a luminescence measuring device is provided within the image field scanner (22,31) with which alternatively or simuntaneously with the measurement of the reflected scanning light the distribution of UV-excitation induced luminescence radiation is measurable and displayable.

17. Apparatus according to claim 16, characterized in that a filter (82) is arranged in the branch of the reference beam path leading to the detector (19) of the first scanning device (31) or is insertable into this branch of the beam path, that suppresses the UV-component of the laser light but is transparent to the luminescence radiation.

18. Apparatus according to any of the preceding claims, characterised in that at least one polarisation adjusting member (84) is provided with which different definite polarisation states of the light beam (24,26) used for scanning the object under examination can be set up, and an exit polariser (86,87) is provided in the detector arrangement which is used as an analyser, which is adjusted or adjustable to a definite polarisation state of the light reflected from the object under examination.

19. Apparatus according to claim 18, characterised in that the entry polariser (84) and the exit polariser (86) are adjusted or adjustable to the same polarisation state.

20. Apparatus according to claim 18 or claim 19, characterised in that the detector arrangement (19, 19') produces a characteristic output signal combination for the polarisation state of the light reflected from the object under examination.

**Revendications**

1. Appareil destiné à visualiser des zones surfaciques et/ou sous forme de tranches minces de l'oeil humain , notamment des sections antérieures de celui-ci, comportant un dispositif d'exploration optique à commande électrique, qui correspond à un réseau de lignes et d'intervalles comme une trame de télévision normalisée courante et éclaire successivement les éléments d'image correspondant à la division du réseau en même temps qu'il mesure l'intensité de la lumière dispersée et/ou réfléchie par les éléments d'image respectivement éclairés pour former un dispositif de détection photoélectrique, et comportant également un dispositif qui extrait du faisceau de rayons indicateurs de la lumière réfléchie sur la cornée et/ou le film de larmes, un laser étant prévu dans le cadre du dispositif d'exploration comme source lumineuse, laser dont le courant lumineux de sortie peut être focalisé sur des sections transvesales du foyer linéaire de moins de 0,5 μm de diamètre, caractérisé en ce qu'on prévoit pour focaliser le faisceau lumineux laser (23,24',26') sur la zone à examiner (16) un objectif de microscope (17), qui procure une focalisation de la lumière d'examen sur la zone à examiner avec une faible profondeur de champ correspondant à l'épaisseur de la tranche de la zone à examiner, en ce que le faisceau des rayons indicateurs est formé par une partie du faisceau des rayons d'éclairage et/ou d'exploration, dont on peut séparer au moyen d'un agencement de division des rayons (48) une partie de la lumière réfléchie en direction de la source lumineuse (22), pour mesurer l'intensité, et en ce qu'on prévoit entre le dispositif de division des rayons (48) et l'agencement de détection (19) un sténopé (53) dont le foyer se trouve dans le plan focal (16) de l'objectif à microscope (17), vu dans le sens de la diffusion de la lumière réfléchie, sténopé par lequel ne peut passer que de la lumière qui provient de la zone de profondeur de champ de la représentation procurée par l'objectif de microscope (17).

2. Appareil selon la revendication 1, caractérisé en ce que sur le trajet du faisceau lumineux se trouve un miroir actif (57) dont le pouvoir réfringent peut être commandé électroniquement, la distance entre les plans d'examen (16, 16',16'') et le plan principal de l'objectif de microscope (17) pouvant être modifiée grâce à la commande électronique.

3. Appareil selon la revendication 2, caractérisé en ce que le miroir actif (57) est disposé, dans le cadre d'une optique de conformation des rayons (27) prévue pour adapter la section transversale du faisceau lumineux d'exploration à la pupille d'entrée (47) de l'objectif de microscope (17) et comportant une optique d'entrée télécentrique (59,61) et une optique de sortie télécentrique (62,63), dans un plan de pupille (47') correspondant au plan de la pupille d'entrée (47) de l'objectif de microscope (17).

4. Appareil selon l'une des revendications précédentes, caractérisé en ce qu'on prévoit une mémoire d'images (21) commandée en synchronisme avec la commande de lignes et d'intervalles du faisceau lumineux balayant l'objet à examiner, mémoire dans laquelle on peut mémoriser les valeurs d'intensité de la lumière réfléchie mesurées pour les éléments d'image individuels d'un plan d'examen (16,16',16'') et ce pour une multiplicité de tels plans d'examen.

5. Appareil selon l'une des revendications précédentes, caractérisé en ce qu'on prévoit un modulateur (56,56',56'') électro-optique ou acousto-optique, grâce à la commande duquel, en synchronisme avec le rythme de mémorisation des signaux de sortie du détecteur dans la mémoire d'images (21), on peut faire varier l'intensité du courant lumineux d'exploration (23,24',26').

6. Appareil selon l'une des revendications précédentes, caractérisé en ce qu'on prévoit un dispositif d'exploration supplémentaire (64) avec lequel on peut saisir la répartition spatiale d'intensité (fonction d'images ponctuelles) de la lumière réfléchie par un élément d'image du champ d'examen (16).

7. Appareil selon la revendication 6, caractérisé en ce qu'on prévoit un dispositif de comparaison qui, à partir de la comparaison de la répartition d'intensité mesurée avec une répartition d'intensité idéale attendue d'après la conception de l'optique de représentation, émet des signau: de réglage pour commander le miroir actif (57) dans le sens d'une compensation de défauts de représentation.

8. Appareil selon la revendication 6 ou la revendication 7, caractérisé en ce qu'on prévoit, pour déterminer la fonction d'images ponctuelles (fonction PS) un certain nombre de récepteurs photoélectriques disposés en matrice, notamment une matrice de diodes bidimensionnelles (73) à la suite de laquelle est montée une unité de calcul (74), qui fournit, à partir du traitement des signaux de sortie de la matrice de diodes (73) caractéristiques de l'intensité, les signaux de réglage nécessaires pour commander le miroir actif (57) dans le sens d'une compensation de défauts de représentation.

9. Appareil selon l'une des revendications précédentes 6 à 8, caractérisé en ce que la lumière arrivant dans le second dispositif d'exploration (64) est séparée du courant lumineux de sortie (23,24,26) de la source de lumière laser (22) au moyen d'un miroir (70) partiellement transparent, l'intensité du courant lumineux (23'',24'',26'') arrivant dans l'autre dispositif d'exploration (64) n'étant que de 1/10 à 1/5 de l'intensité du courant de lumière laser de sortie (23,24,26).

10. Appareil selon l'une des revendications précédentes 6 à 9, caractérisé en ce que l'intensité de la lumière arrivant dans l'autre dispositif d'exploration (64) peut être réglée au moyen d'un dispositif de modulation propre.

11. Appareil selon la revendication 9 et la revendication 10, caractérisé en ce que la lumière arrivant dans l'autre dispositif d'exploration peut être séparée du courant lumineux de sortie de la source lumineuse (22) au moyen d'un miroir partiellement transparent disposé entre la source lumineuse (22) et le premier modulateur (56), le dispositif de modulation de l'autre dispositif d'exploration (64) étant monté à la suite du miroir partiellement transparent.

12. Appareil selon l'une des revendications précédentes en liaison avec la revendication 6, caractérisé en ce qu'on peut amener dans l'autre dispositif d'exploration (64) un courant lumineux d'essai ayant une répartition spectrale à large bande de la lumière d'essai, et en ce qu'on prévoit un spectromètre (78) avec lequel on peut saisir la répartition d'intensité spectrale de la lumière projetée dans la branche de sortie de l'autre dispositif d'exploration (64).

13. Appareil selon la revendication 12, caractérisé en ce que le spectromètre prévu dans la branche d'essai de la lumière blanche du dispositif (10) comporte comme dispositif détecteur une multiplicité de détecteurs photo-électriques disposés en ligne,

14. Appareil selon l'une des revendications précédentes, caractérisé en ce que la source lumineuse (22) prévue pour explorer le champ d'examen (16) a au moins deux plages d'émission de longueurs d'ondes nettement différentes $\lambda_1$ et $\lambda_2$, l'une des plages d'émission de longueur d'onde centrale $\lambda_1$ se trouvant dans la plage UV proche (entre 300 nm et 400 nm) et l'autre plage d'émission de longueur d'onde centrale $\lambda_2$ se trouvant dans la plage visible du spectre ( $\lambda$ supérieur à 400 nm).

15. Appareil selon la revendication 14, caractérisé en ce qu'on prévoit comme source lumineuse (22) un laser He-Cd, qui émet des longueurs d'onde $\lambda_1$ = 325 nm et $\lambda_2$ = 440 nm.

16. Appareil selon la revendication 14 ou la revendication 15, caractérisé en ce qu'on prévoit dans le cadre du dispositif d'exploration du champ d'image (22,31) un dispositif de mesure de luminescence avec lequel on peut saisir et représenter, simultanément avec la saisie de la lumière d'exploration réfléchie ou en alternance avec cette saisie, également la répartition du rayonnement luminescent induit par une excitation UV en provenance de l'objet à examiner.

17. Appareil selon la revendication 16, caractérisé en ce qu'on dispose ou qu'on peut introduire dans la branche du trajet du faisceau indicateur conduisant au détecteur (19) du premier dispositif d'exploration (31) un filtre (82), qui supprime les composantes UV de la lumière laser, mais laisse passer le rayonnement luminescent.

18. Appareil selon l'une des revendications précédentes, caractérisé en ce qu'on prévoit au moins un organe de réglage de polarisation (84) avec lequel on peut régler différents états de polarisation définis du courant lumineux (24,26) utilisé pour explorer l'objet à examiner et en ce qu'on prévoit dans le cadre de l'agencement de détecteurs un polariseur de sortie (86;87) utilisé comme analyseur, qui, de son côté, est réglé, ou peut être réglé sur un état de polarisation défini de la lumière renvoyée par l'objet à examiner.

19. Appareil selon la revendication 18, caractérisé en ce que le polariseur d'entrée (94) et le polariseur de sortie (86) sont réglés ou peuvent être réglés sur le même état de polarisation.

20. Appareil selon la revendication 18 ou la revendication 19, caractérisé en ce que l'agencement de détecteurs (19,19') produit une combinaison de signaux de sortie caractéristique pour l'état de polarisation de la lumière renvoyée par l'objet à examiner.

Fig.1

gemessene Intensität $I_m(u,v)$

ersetze $I$ durch $I_m$
$I \longrightarrow I_m$

Brenn - (Fourier -) ebene

$\mathscr{F}$

$\sqrt{I(u,v)} \cdot \exp i \gamma(u,v)$

$\mathscr{F}^{-1}$

$\sqrt{I_m(u,v)} \cdot \exp i \gamma(u,v)$

Fig. 2

Start $\quad \exp i K \varphi_0(x,Y)$

$\exp i K \varphi_{1...n}(x,Y)$

$R'(x,Y) = \begin{cases} 1, & x^2+Y^2 \leq 1 \\ 0, & x^2+Y^2 > 1 \end{cases}$ wenn:

$R'(x,Y) \cdot \exp i K \varphi_{1...n}(x,Y)$

Pupillenebene

Fig.3